# EUROPEAN PATENT APPLICATION

(11) **EP 4 467 172 A1**
(43) Date of publication of application: **27.11.2024**
(21) Application number: 23756692.2
(22) Date of filing: 20.02.2023
(51) Int. Cl.: A61M 5/142

(54) **FLUID CONVEYING PUMP DEVICE**

(30) Priority: 21.02.2022 KR 20220022215; 21.02.2022 KR 20220022253; 21.02.2022 KR 20220022270
(71) Applicant: Caremedi Co., Ltd., Seoul 04107 (KR); Sogang University Research & Business Development Foundation, Seoul 04107 (KR)
(72) Inventor: SHIN, Woonsup, Seoul 04065 (KR); ZHU, Enhua, Seoul 04109 (KR); LEE, Junghyun, Seoul 03916 (KR)
(74) Representative: Manitz Finsterwald Patent- und Rechtsanwaltspartnerschaft mbB
(86) International application number: PCT/KR2023/002387
(87) International publication number: WO 2023/158280

(57) **Abstract**

Embodiments of the present disclosure provide a fluid transfer pump device in which a plurality of components constituting the fluid transfer pump device form a stack structure. Embodiments of the present disclosure provide an electroosmotic pump having an easily couplable structure and a power supply device for the electroosmotic pump. Embodiments of the present disclosure provide a fluid transfer device including an inflow path and an outflow path capable of increasing transfer efficiency of a fluid and preventing blockage of a flow path.

## Description

### BACKGROUND

The present disclosure relates to a fluid transfer pump device, and more particularly, to a fluid transfer pump device for drug injection.

Depending on the type, purpose of treatment, method, and so on, drugs may be injected into the body in various forms, such as oral, subcutaneous, and intravenous administration. A drug injector using a drug pump may automatically inject drugs into the body at a desired speed and dose at a required time. Accordingly, the drug injector using a drug pump may be used not only in hospitals or in patients' daily living environments, but also in various forms.

An insulin pump, commonly called an insulin injector, is a medical device that supplies insulin into the body accurately at a set time from the outside to control blood sugar, similar to a pancreas, for diabetic patients who do not secrete insulin or secrete only a small amount of insulin.

The insulin pump is used for insulin-dependent diabetic patients, and insulin pump technology has problems requiring miniaturization and precision administration, and much research is currently being conducted to solve the problems.

Metering pumps applicable to drug infusion devices, such as insulin pumps, are pumps that transfer fluid at a constant rate per unit time and may be divided into diaphragm pumps (membrane pumps), gear pumps, peristaltic pumps, and syringe pumps depending on operating principles, and are used in a wide range of industrial fields.

Drug injection may be performed continuously or intermittently depending on the amount and speed of injection. Accurately and stably injecting the drug is the most important part for effective treatment and user safety.

In particular, the biggest reason for not accurately injecting the drug by insulin pumps is that solid substances are formed at the tip of the cannula, needle, catheter, or so on where drug is injected and the part where the body fluid comes into contact, which interferes with the injection of drug.

Therefore, a device that may detect whether the drug is being injected at a desired rate or in the desired amount is essential for safe and stable injection, and drug injection technology that prevents such problems from occurring at the source is important.

### SUMMARY

A fluid transfer pump device according to an embodiment of the present disclosure may increase a fluid transfer efficiency and detect blockage of a flow path.

In addition, an electroosmotic pump according to an embodiment of the present disclosure may easily perform the coupling with a power supply device and increase a contact area with the power supply device.

In addition, a fluid transfer device according to an embodiment of the present disclosure may increase a fluid transfer efficiency and reduce a dead space of a fluid.

However, technical problems to be solved by the present embodiment are not limited to the technical problems described above, and there may be other technical problems.

According to a first aspect of the present disclosure, a fluid transfer pump device includes a pressure generating unit configured to alternately and electrochemically generate positive pressure and negative pressure, a first isolation unit coupled to a first side of the pressure generating unit, a second isolation unit coupled to a second side of the pressure generating unit, and a transfer chamber unit coupled to the second isolation unit on an opposite side of the pressure generating unit.

Also, the pressure generating unit, the first isolation unit, the second isolation unit, and the transfer chamber unit may be stacked in one direction and integrally coupled to each other.

Also, the pressure generating unit, the first isolation unit, the second isolation unit, and the transfer chamber unit may be formed and aligned to have a rectangular cross-sectional outer shape with respect to a stacking direction.

Also, the pressure generating unit may be an electroosmotic pump.

Also, the electroosmotic pump may include a membrane configured to allow movement of a fluid, and a first electrode and a second electrode which have a plate shape and are respectively stacked on and respectively coupled to both sides of the membrane.

Also, the first isolation unit may include a first isolation member frame stacked on and coupled to the pressure generating unit, and a flexible first isolation unit coupled to an inside of the first isolation member frame and sealing the pressure generating unit.

Also, the first isolation member frame may be coupled to the first isolation member by using at least one of a thermal fusion method, an ultraviolet ray bonding method, and an ultrasonic fusion method.

Also, the fluid transfer pump device may further include a monitoring unit coupled to the first isolation unit on the opposite side of the pressure generating unit.

Also, the monitoring unit may include a monitoring chamber frame stacked on and coupled to the first isolation unit, a monitoring chamber formed inside the monitoring chamber frame, and a pressure sensor configured to measure pressure of the monitoring chamber.

Also, a pressure sensor coupling groove communicating with the monitoring chamber may be formed in the monitoring chamber frame, and the pressure sensor may be inserted into and fixed to the pressure sensor coupling groove.

Also, the second isolation unit may include a second isolation member frame stacked on and coupled to the pressure generating unit, and a second isolation member that is flexible and coupled to an inside of the second isolation member frame and seals the pressure generating unit.

According to a second aspect of the present disclosure, an electroosmotic pump includes a membrane configured to allow movement of a fluid, and a first electrode and a second electrode respectively provided on both sides of the membrane, wherein the membrane, the first electrode, and the second electrode are stacked in a plate shape and coupled to each other, and the first electrode and the second electrode respectively include electrode portions corresponding to the membrane, and terminal portions integrally extended and protruded to one side of each of the electrode portions.

Also, the terminal portion may have a plate shape.

Also, the electroosmotic pump may further include a power supply unit connected to the terminal portions, wherein the power supply unit includes a first connector and a second connector respectively connected to the terminal portions, and a support member fixedly supporting the first connector and the second connector.

Also, the first connector and the second connector may be fixed on the support member at a preset distance matching a distance between the first electrode and the second electrode and may be respectively and slidably coupled to the first electrode and the second electrode.

Also, each of the first connector and the second connector may include a first leg portion and a second leg portion formed to elastically support both sides of each of the terminal portions.

Also, each of the first leg portion and the second leg portion may have a folded portion formed to elastically support one of the terminal portion and to expand an interval between the first leg portion and the second leg portion toward a side surface to which the one of the terminal portions is coupled.

Also, the electroosmotic pump may further include a third leg portion formed to be folded from at least one of the first leg portion and the second leg portion toward the one of the terminal portions to expand a contact surface with the one of the terminal portions.

According to a third aspect of the present disclosure, a fluid transfer device includes an isolation member which is flexible and to which positive pressure and negative pressure are alternately transferred, and a transfer chamber unit coupled to the isolation member, wherein the transfer chamber portion includes a transfer chamber frame defining an outer shape of the transfer chamber unit, and a transfer chamber which is formed concavely in the transfer chamber frame to face the isolation member and which a transfer target fluid is input to and output from.

Also, the fluid transfer chamber may include an upper portion that is open toward the isolation member and is sealed by being coupled to the isolation member, a bottom portion formed on an opposite side of the isolation member, and a peripheral portion formed between the upper portion and the bottom portion.

Also, a cross-sectional area of the upper portion may be greater than a cross-sectional area of the bottom portion, and the peripheral portion has an inclined surface.

Also, when the positive pressure is applied to the transfer chamber through the isolation member, the isolation member may be deformed toward the bottom portion to correspond to a shape of the inclined surface and discharges the transfer target fluid.

Also, when the negative pressure is applied to the transfer chamber through the isolation member, the isolation member may be deformed toward an opposite side of the bottom portion and sucks the transfer target fluid.

Also, an inlet to which the transfer target fluid is input and an outlet through which the transfer target fluid is output from the transfer chamber may be formed in the peripheral portion.

Also, the inlet and the outlet may be formed to face each other in the peripheral portion.

Also, the fluid transfer device may further include an inflow path extending from the inlet, and an outflow path extending from the outlet.

Also, the inflow path and the outflow path may be formed to be inclined to face outward respectively with respect to the inlet and the outlet.

Also, the inflow path and the outflow path may increase in cross-sectional area as a distance from the transfer chamber increases.

A fluid transfer pump device according to an embodiment of the present disclosure may increase a transfer efficiency of a fluid and detect blockage of flow paths.

In addition, an electroosmotic pump according to an embodiment of the present disclosure may easily perform coupling to a power supply device and may increase a contact area with the power supply device.

In addition, a fluid transfer device according to an embodiment of the present disclosure may increase a transfer efficiency of a fluid and reduce a dead space of the fluid.

### BRIEF DESCRIPTION OF THE DRAWINGS

Embodiments of the inventive concept will be more clearly understood from the following detailed description taken in conjunction with the accompanying drawings in which:
FIG. 1 is an exploded view of a fluid transfer pump device according to an embodiment;
FIG. 2 is a front view of a fluid transfer pump device according to an embodiment;
FIG. 3 is example views of operations of a fluid transfer pump device according to an embodiment;
FIG. 4 is an exploded view of an electroosmotic pump according to an embodiment;
FIG. 5 is an exploded view of an electroosmotic pump and an isolation unit according to an embodiment;
FIG. 6 is a structural view of a first isolation unit according to an embodiment;
FIG. 7 is a cross-sectional view of a first isolation unit according to an embodiment;
FIG. 8 is a structural view of a second isolation unit according to an embodiment;
FIG. 9 is a cross-sectional view of a second isolation unit according to an embodiment;
FIG. 10 is a plan view of a second isolation unit according to an embodiment;
FIG. 11 is example views of coupling of a power supply unit and a connector according to an embodiment;
FIG. 12 is a side view of a power supply unit according to an embodiment;
FIG. 13 is a detailed view of a connector according to an embodiment;
FIG. 14 is a side view of a power supply unit according to an embodiment.
FIG. 15 is a detailed view of a connector according to an embodiment;
FIG. 16 is a structural view of a transfer chamber unit according to an embodiment;
FIG. 17 is an exploded view of a transfer chamber unit and a second isolation unit according to an embodiment;
FIG. 18 is example views of deformation of a second isolation member according to an embodiment;
FIG. 19 is a cross-sectional view of a transfer chamber unit according to an embodiment;
FIG. 20 is an example view of deformation of a transfer chamber unit according to an embodiment;
FIG. 21 is an example view of deformation of a transfer chamber unit according to an embodiment;
FIG. 22 is an exploded view of a valve unit and a transfer path unit according to an embodiment;
FIG. 23 is a cross-sectional view of a transfer chamber unit and a transfer path unit according to an embodiment;
FIG. 24 is an exploded view of a monitoring unit according to an embodiment; and
FIG. 25 is deformation of a first isolation member according to an embodiment.

### DETAILED DESCRIPTION OF THE EMBODIMENTS

Hereinafter, embodiments of the present disclosure will be described in detail with reference to the accompanying drawings such that those skilled in the art in which the present disclosure belongs may easily practice the present disclosure. However, the present disclosure may be implemented in many different forms and is not limited to the embodiments described herein. In addition, in order to clearly describe the present disclosure, parts irrelevant to the description are omitted in the drawings, and similar reference numerals are attached to similar parts throughout the specification.

When it is described that a portion is "connected" to another portion throughout the specification, this includes not only a case where the portion is "directly connected" to another portion but also a case where the portion is "electrically connected" to another portion with another component therebetween. In addition, when it is described that a portion "includes" a certain component, this means that the portion may further include another component without excluding another component unless otherwise stated.

In addition, the attached drawings are only intended to easily understand the embodiments disclosed in the present specification, and the technical ideas disclosed in the present specification are not limited by the attached drawings and should be understood to include all modifications, equivalents, or substitutes included in the scope of the present disclosure and technology.

Terms including ordinal numbers, such as first, second, and so on, may be used to describe various components, but the components are not limited by the terms. The terms are used only for the purpose of distinguishing one component from another component.

When a component is referred to as being "connected" or "coupled" to another component, it should be understood that the component may be directly connected or coupled to another component, but that there may be other components therebetween. In contrast to this, when a component is referred to as being "directly connected" or "directly coupled" to another component, it should be understood that there are no other components therebetween.

singular expressions include plural expressions, unless the context clearly indicates otherwise.

In the present specification, it should be understood that terms, such as "comprise", "include", and "have", are intended to designate the presence of features, numbers, steps, operations, configuration elements, components, or combinations thereof described in the specification and do not exclude in advance the presence or addition of one or more other features, numbers, steps, operations, configuration elements, components, or combinations thereof.

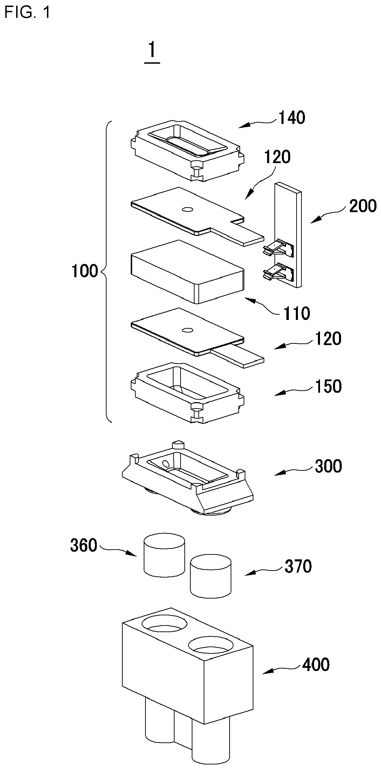

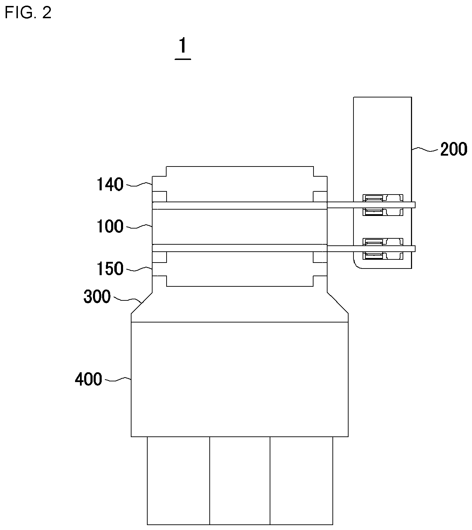

Hereinafter, a configuration of a fluid transfer pump device 1 according to an embodiment will be described with reference to FIGS. 1 and 2.

FIG. 1 is an exploded view of the fluid transfer pump device 1 according to the embodiment.

Referring to FIG. 1, the fluid transfer pump device 1 includes a pressure generating unit 100 and a power supply unit 200. In addition, the fluid transfer pump device 1 may include a transfer chamber unit 300 and a transfer path unit 400 through which a first fluid, which is a transfer target fluid, moves. Additionally, the fluid transfer pump device 1 may further include a monitoring unit 500 that is separately provided without being coupled to a path through which the first fluid moves and detects a normal operation of the fluid transfer pump device 1 and blockage of a flow path.

The fluid transfer pump device 1 may mean a device that inhales and discharges a drug and a pumping device that transfers the first fluid. However, the use of the fluid transfer pump device 1 described in the present specification is not limited to the transfer of drugs and may be used to inhale and discharge various fluid-type materials.

The pressure generating unit 100 is a general term for a device that generates pressure for transferring the first fluid which is a transfer target fluid. The pressure generating unit 100 may generate positive pressure or negative pressure. The positive pressure and negative pressure refer to a direction of pressure generated for changing a direction of movement of a fluid.

Accordingly, the negative pressure refers to a direction of pressure for inhaling the first fluid, and the positive pressure refers to a direction of pressure for discharging the first fluid. The fluid transfer pump device 1 may inhale the drug stored in a drug storage unit and inject the drug into the body according to the positive pressure and negative pressure generated by the pressure generating unit 100.

When an electroosmotic pump is used as the pressure generating unit 100, the pressure generating unit 100 includes a second fluid, a membrane 110 through which a second fluid passes, a first electrode 120, and a second electrode 120. The first electrode 120 and the second electrode 120 may be respectively arranged on side surfaces of the membrane 110.

Specifically, the first electrode 120 and the second electrode 120 may be respectively arranged on opposite side surfaces of the membrane 110, or on planes perpendicular to the direction of pressure. The first electrode 120 and the second electrode 120 may receive power for generating pressure from the power supply unit 200.

In addition, the pressure generating unit 100 further includes isolation units 140 and 150 respectively stacked on and coupled to the first electrode 120 and the second electrode 120. The isolation units 140 and 150 may respectively include a first isolation unit 140 coupled to one side of the pressure generating unit 100 and a second isolation unit 150 coupled to the other side of the pressure generating unit 100.

The isolation units 140 and 150 may respectively include isolation member frames 141 and 151 stacked on and coupled to the pressure generating unit 100 and flexible isolation members 142 and 152 coupled to the insides of the isolation member frames 141 and 151 to seal the pressure generating unit 100.

Accordingly, the isolation unit 150 prevents the second fluid in the pressure generating unit 100 and the first fluid, which is a transfer target fluid from mixing together. In addition, the isolation members 142 and 152 are deformed in response to the pressure generated by the pressure generating unit 100, and accordingly, the pressure may be transferred to the transfer chamber unit 300, the transfer path unit 400, and the monitoring unit 500.

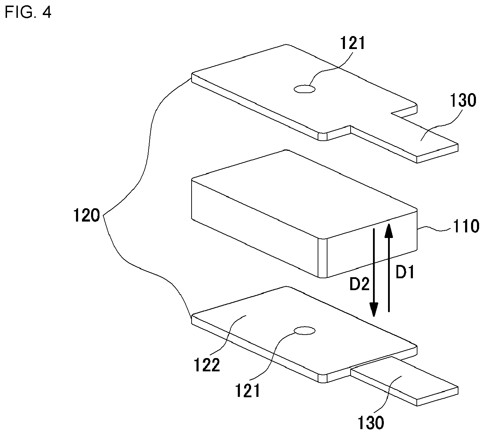

Specific configurations of the pressure generating unit 100 and the power supply unit 200 will be described in detail below with reference to FIGS. 4 to 15.

The fluid transfer pump device 1 includes the transfer chamber unit 300 coupled to the second isolation unit 150 on an opposite side of the pressure generating unit 100. Accordingly, the pressure generating unit 100, the first isolation unit 140, the second isolation unit 150, and the transfer chamber unit 300 are coupled to each other in a stacked manner.

The transfer chamber unit 300 transfers the pressure generated by the pressure generating unit 100 to the first fluid. To this end, the transfer chamber unit 300 includes a transfer chamber frame 310 stacked on and coupled to the second isolation unit 150, a transfer chamber 320 formed inside the transfer chamber frame 310 to receive the transfer target fluid, an inflow portion to which a fluid is input, and an outflow portion from which the fluid is discharged to the transfer chamber 320.

In addition, the fluid transfer pump device 1 further includes the transfer path unit 400 coupled to the transfer chamber unit 300 on an opposite side of the second isolation unit 150. The transfer path unit 400 includes a transfer path frame 410 stacked on and coupled to the transfer chamber unit 300, an inflow route 430 through which the first fluid is input to the transfer chamber unit 300 side, and an outflow route 440 through which the first fluid is output from the transfer chamber unit 300 side.

In this case, the inflow route 430 and the outflow route 440 may be formed in a stacking direction of the fluid transfer pump device 1 or may be formed perpendicularly to the stacking direction.

The fluid transfer pump device 1 may further include valve units 360 and 370 that allows or blocks the flow of the first fluid. The valve units 360 and 370 may include an inflow valve 360 that allows the input of the first fluid to the transfer chamber unit 300 and blocks the output of the first fluid, and an outflow valve 370 that allows the output of the first fluid to the transfer chamber unit 300 and blocks the input of the first fluid. Accordingly, a pair of valve coupling grooves 420 which the inflow valve 360 and the outflow valve 370 are safely placed on and fixed to may be formed in the transfer path unit 400.

The fluid transfer pump device 1 may further include the monitoring unit 500 that is stacked on and coupled to the first isolation unit on an opposite side of the pressure generating unit 100. The monitoring unit 500 may be coupled to an opposite side of the transfer chamber unit 300 and the transfer path unit 400 with respect to the pressure generating unit 100. Accordingly, the monitoring unit 500 may detect whether the fluid transfer pump device 1 normally operates without coming into contact with or being coupled to a path through which the first fluid moves.

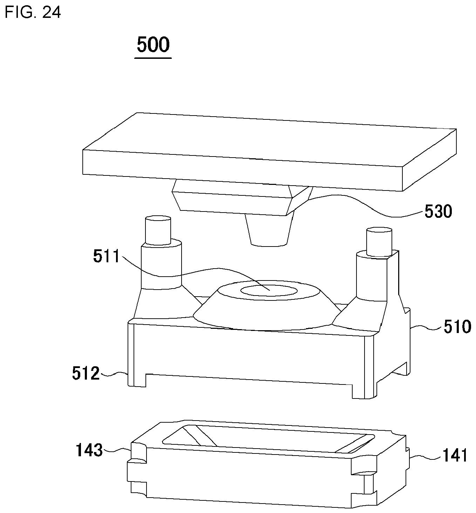

The specific configuration of the monitoring unit 500 will be described in detail below with reference to FIGS. 24 to 25.

The fluid transfer pump device 1 is configured such that two or more of the pressure generating unit 100, the first isolation unit 140, the second isolation unit 150, the transfer chamber unit 300, the transfer path unit 400, and the monitoring unit 500 are stacked in one direction and coupled as one piece. As a plurality of components included in the fluid transfer pump device 1 are stacked in one direction, the components may be easily coupled to each other, and a method of manufacturing the fluid transfer pump device 1 may be simplified.

In addition, the pressure generating unit 100, the first isolation unit 140, the second isolation unit 150, the transfer chamber unit 300, the transfer path unit 400, and the monitoring unit 500 included in the fluid transfer pump device 1 may be aligned to have a rectangular cross-sectional outer shape with respect to the stacking direction.

As the pressure generating unit 100, the first isolation unit 140, the second isolation unit 150, the transfer chamber unit 300, the transfer path unit 400, and the monitoring unit 500 may be manufactured in a thin shape by being designed in a rectangular shape. In addition, components of the fluid transfer pump device 1 are configured to have a rectangular cross-sectional outer shape with respect to the stacking direction, and accordingly, positions of the components may be easily aligned when the components are combined with each other.

The components included in the fluid transfer pump device 1 are designed to have constant standards, and accordingly, an outer shape of the fluid transfer pump device 1 may be a rectangular parallelepiped shape. That is, the pressure generating unit 100, the first isolation unit 140, the second isolation unit 150, the transfer chamber unit 300, the transfer path unit 400, and the monitoring unit 500 included in the fluid transfer pump device 1 are designed to have constant standards, and accordingly, the outer shape of the fluid transfer pump device 1 may be a rectangular parallelepiped shape.

Accordingly, outer shapes of the components included in the fluid transfer pump device 1 may be matched to each other, and the fluid transfer pump device 1 may be designed to reduce shapes of protrusions. By reducing the shape of a protrusion of the fluid transfer pump device 1, the volume of the fluid transfer pump device 1 may be reduced.

In particular, the fluid transfer pump device 1 may be designed to have a rectangular solid shape having a height of 5 to 10 mm, a width of 10 to 15 mm, and a length of 15 to 30 mm to be applied to a miniaturized drug injector. By configuring the fluid transfer pump device 1 in a single module shape having a constant standard, the fluid transfer pump device 1 may be used for various types of drug injection devices.

By designing the protrusion of the fluid transfer pump device 1 in a reduced shape and designing an outer shape of the protrusion of the fluid transfer pump device 1 in a rectangular solid shape, the fluid transfer pump device 1 may be easily coupled to and compatible with other components in a housing of a drug injection device such as an insulin patch.

In addition, by designing the fluid transfer pump device 1 to have a one-way stack structure, the stack structure of the fluid transfer pump device 1 may be easily modified or partially replaced with other components depending on the purpose of use, the type of used fluid, the type and standard of a drug injection device, and so on. Standards of the fluid transfer pump device 1 are not limited thereto and may be changed depending on a stack structure, a change in the stack structure, and the type and standard of a drug injection device to be applied.

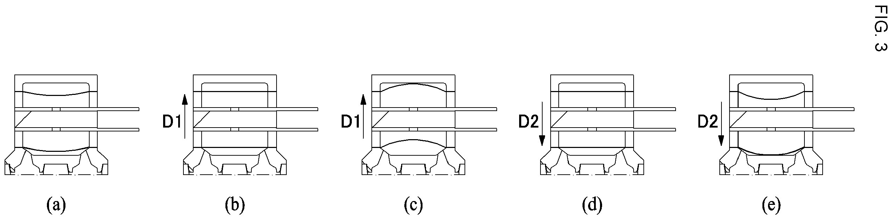

Hereinafter, an operation of the fluid transfer pump device 1 according to the embodiment will be described with reference to FIG. 3.

FIG. 3 is example views of operations of the fluid transfer pump device according to the embodiment.

FIG. 3(a) illustrates a case where no pressure is generated in the pressure generating unit 100. As illustrated in FIG. 3(a), when no pressure is generated in the pressure generating unit 100, the flexible isolation members 142 and 152 are stretched in a direction of gravity. That is, when no pressure is generated, the isolation member 142 (may be referred to as a first isolation member 142) and the isolation member 152 (may be referred to as a second isolation member 152) may each have a parabolic shape that is bent downward.

FIG. 3(b) and 3(c) illustrate a case where negative pressure is generated in the pressure generating unit 100. When pressure is generated in a direction D1 from the pressure generating unit 100 as illustrated in FIG. 3(b), deformation of the flexible isolation members 142 and 152 occurs. Accordingly, the first isolation member 142 and the second isolation member 152 are deformed in the direction D1. Accordingly, as illustrated in FIG. 3(c), the first isolation member 142 and the second isolation member 152 are deformed into a first shape. The first shape means a shape in which isolation members are deformed in response to the negative pressure.

The first shape is a shape in which the first isolation member 142 or the second isolation member 152 is raised. Accordingly, the first shape may mean that the isolation member 142 or 152 is deformed in a parabolic shape which is bent upward.

When the second isolation member 152 is deformed in the first shape as illustrated in FIG. 3(b), the inflow valve 360 is opened. In addition, when the second isolation member 152 is deformed in the first shape, the outflow valve 370 is closed. Accordingly, the first fluid, that is a transfer target fluid, is input to the transfer chamber 320 along the inflow path 330.

The monitoring unit 500 may detect whether the fluid transfer pump device 1 operates normally and whether a flow path is blocked by measuring a change in pressure or shape according to deformation of the first isolation member 142 to the first shape.

FIGS. 3(d) and 3(e) illustrate a case where positive pressure is generated in the pressure generating unit 100. When pressure is generated in a direction D2 from the pressure generating unit 100 as illustrated in FIGS. 3(d) and 3(e), the flexible isolation members 142 and 152 are deformed. Accordingly, the first isolation member 142 and the second isolation member 152 are caused to be deformed in the direction D2. Accordingly, as illustrated FIG. 3(e), the first isolation member 142 and the second isolation member 152 are deformed in a second shape. The second shape means a shape in which the isolation members 142 and 152 are deformed in response to the positive pressure.

The second shape is a shape in which the first isolation member 142 or the second isolation member 152 is lowered. The second shape may mean a shape symmetrical to the first shape. Accordingly, the second shape may mean that the insulation members 142 or 152 is deformed in a parabolic shape which is bent downward.

When the first isolation member 142 or the second isolation member 152 is deformed in the second shape in response to the positive pressure, a deformation rate of the second shape may be greater than a basic shape of the flexible isolation members 142 and 152 illustrated in FIG. 3(a). That is, when positive pressure occurs, bending of the isolation members 142 and 152 may be greater than the basic shape.

When the second isolation member 152 is deformed in the second shape as illustrated in FIG. 3(e), the inflow valve 360 is closed. In addition, when the second isolation member 152 is deformed in the second shape, the outflow valve 370 is opened. Accordingly, the first fluid in the transfer chamber 320 is output along the outflow path 340.

The monitoring unit 500 may detect whether the fluid transfer pump device 1 operates normally and whether a flow path is blocked by measuring a change in pressure or shape according to deformation of the first isolation member 142 to the second shape.

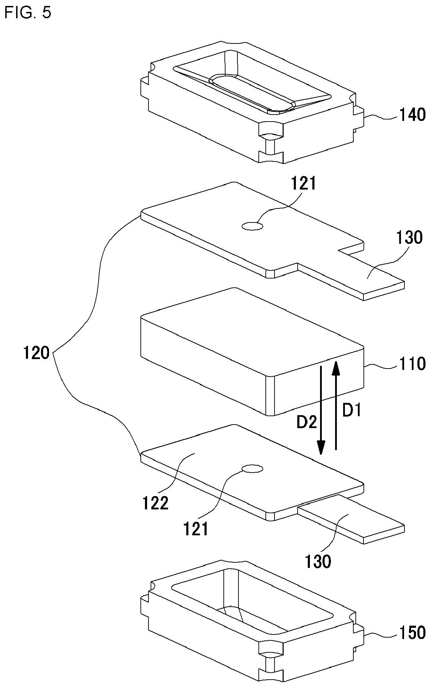

Hereinafter, a structure of an electroosmotic pump according to an embodiment will be described with reference to FIGS. 4 and 5.

The pressure generating unit 100 alternately generates positive pressure and negative pressure. The pressure generating unit 100 may include all pumps and pressure generating devices, which may alternately generate positive pressure and negative pressure, such as an electroosmotic pump, a diaphragm pump that sucks and discharges the first fluid by the movement of a diaphragm, a pump using a motor, and a hydraulic pump.

According to one example, when the pressure generating unit 100 includes an electroosmotic pump, the electroosmotic pump may include at least one component that moves the first fluid by using an electrochemical reaction but is not limited thereto.

The electroosmotic pump generates positive pressure and negative pressure by using an electroosmotic principle. The electroosmotic pump has a pump structure that uses the movement of the second fluid in the electroosmotic pump due to an electroosmotic phenomenon that occurs when a voltage is applied to electrodes of both ends of a capillary or porous membrane. Accordingly, the electroosmotic pump may move a fluid by using a simple electrochemical reaction. Therefore, the electroosmotic pump does not require a mechanical configuration, thereby being operated noiselessly. In addition, the intensity of pressure may be effectively adjusted in proportion to a voltage applied to the electroosmotic pump.

The electroosmotic pump may include a membrane 110 and at least one of the first electrode 120 and the second electrode 120. The electroosmotic pump includes the membrane 110 that allows the movement of fluid, and the first electrode 120 and the second electrode 120 of a plate shape which are stacked on and respectively coupled to both sides of the membrane 110. The first electrode 120 and the second electrode 120 may each include an electrode portion 122 corresponding to a membrane region, and a terminal portion 130 that extends and protrudes to one side of the electrode portion 122.

The first electrode 120 and the second electrode 120 may each be composed of an electrode formed of a porous material or an impermeable material, or a base material and an electrode material coated on the base material, and an impermeable base material may be formed with one or more fluid movement passages 121.

The impermeable material may include at least one of a conductive material, a semiconductor material, and a non-conductive material. In this case, the conductive material includes at least one selected from among carbon, nickel, copper, gold, silver, titanium, ruthenium, palladium, zinc, platinum, cobalt, lead, manganese, tin, iridium, iron, aluminum, gold oxide, silver oxide, ruthenium oxide, platinum oxide, lead oxide, iridium oxide, polypyrrole, a derivative of polypyrrole, polyaniline, a derivative of polyaniline, polythiophene, a derivative of polythiophene, and combinations thereof. The semiconductor material includes at least one selected from among Sn, Si, SiC, Ge, Se, AIP, AlAs, AlSb, GaP, GaAs, InP, InAs, InSb, ZnS, ZnSe, ZnTe, CdS, CdSe, CdTe, ZnO, SnO₂, SiO₂, CeO₂, TiO₂, WOs, Fe₂O₃, In₂O₃, CuO, polypyrrole, a derivative of polypyrrole, polyaniline, a derivative of polyaniline, polythiophene, a derivative of polythiophene, prussian blue, iron hexacyanoferrate (FeHCF), copper hexacyanoferrate (CuHCF), cobalt hexacyanoferrate (CoHCF), and nickel hexacyanoferrate (NiHCF), and combinations thereof. The non-conductive material includes polyethylene (PE), polypropylene (PP), polyvinylidene fluoride (PVDF), polyvinylidene chloride (PVDC), ethylenevinylalcohol (EVOH), polyethylene terephthalate (PET), poly(methyl methacrylate) (PMMA), polyolefin, polyamide, polyester, aramid, acrylic, polyethylene oxide, polycaprolactone, polycarbonate, polyurethane (PU), polystyrene, polybenzimidazole (PBI), poly(2-hydroxyethyl methacrylate), It includes at least one selected from poly(ether imide), styrene-butadiene-styrene triblock copolymer (SBS), poly(ferrocenyldimethylsilane), polyimide (PI), and combinations thereof.

The second fluid included in the electroosmotic pump may move through the fluid movement passages 121 of the first electrode 120 and the second electrode 120. Accordingly, the electroosmotic pump moves the second fluid in the electroosmotic pump by using an electrochemical reaction of the first electrode 120 and the second electrode 120, and positive pressure and negative pressure are generated by the movement of the second fluid.

In FIG. 4, the fluid movement passages 121 are illustrated as circles. However, the fluid movement passages 121 of the present disclosure are not limited thereto and may be formed in various shapes, such as a square, triangle, and a diamond shape depending on the type and capacity of the second fluid, a combination form of the membrane 110 and the first electrode 120 and the second electrode 120, a method of manufacturing the first electrode 120 and second electrode 120, and so on.

The electroosmotic pump supplies voltages to the first electrode 120 and the second electrode 120 by alternating polarities of the voltages, and thereby, forward and reverse electrochemical reactions are reversibly generated. As the forward and reverse electrochemical reactions are repeatedly generated, the second fluid in the electroosmotic pump repeatedly performs reciprocating motions. In addition, by the repetitive forward and reverse reversible electrochemical reactions, the first electrode 120 and the second electrode 120 are repeatedly consumed and regenerated.

FIG. 4 illustrates that the membrane 110, the first electrode 120, and the second electrode 120 each have a rectangular or rectangular parallelepiped shape but are not limited thereto and may have various shapes depending on the purpose, size, and coupling form of the fluid transfer pump device 1.

The first electrode 120 and the second electrode 120 may be coupled to a side surface of the membrane 110 that is perpendicular to a direction in which the second fluid moves or a side surface that is perpendicular to a direction in which positive pressure and negative pressure are generated. In addition, sizes of the first electrode 120 and the second electrode 120 are not required to be equal to a size of one side of the membrane 110. The first electrode 120 and the second electrode 120 may each be formed in a plate shape that covers one side of the membrane 110.

In addition, the first electrode 120 and the second electrode 120 according to the embodiment of the present disclosure may each have the terminal portion 130 that protrudes to be coupled to the power supply unit 200. That is, the terminal portion 130 is formed in a plate shape that protrudes and extends integrally to one side of the electrode 120.

Accordingly, the terminal portion 130 has a shape that may be coupled to or come into contact with the power supply unit 200. The terminal portion 130 are not required to be coated with an electrode coating material but may also be selectively coated.

In one example, the membrane 110 may include one of silicon dioxide (SiO₂), barium titanate (BaTiOs), silicon carbide (SiC), silicon nitride (Si₃N₄), and zirconium dioxide (ZrO₂), or a mixture of one or more thereof.

In addition, in one example, the first electrode 120 and the second electrode 120 may each include at least one of a carbon electrode, a polymer electrode, a metal, metal oxide, metal hexacyanoferrate, and so on and may also include a mixture of one or more thereof. In one example, the metal serving as an electrode coating material may include at least one of silver, zinc, lead, manganese, copper, tin, ruthenium, nickel, gold, titanium, palladium, platinum, cobalt, iron, aluminum, iridium, and a combination thereof. Also, the carbon electrode may include at least one of carbon nanotube (CNT), graphene, carbon nanoparticle, fullerene, graphite, and a combination thereof.

In one example, the polymer electrode material serving as an electrode coating material may include at least one of polyaniline, a derivative of polyaniline, polythiophene, a derivative of polythiophene, polypyrrole, a derivative of polypyrrole, polythionine, and a quinone polymer, or a mixture one or more thereof.

In addition, metal oxide may be used as the electrode coating material. The metal oxide may include at least one of, for example, silver/silver oxide (Ag/AgO₂), platinum oxide, lead oxide, manganese oxide (MnOₓ), vanadium oxide (V₂O₅), molybdenum oxide (MoOs), tungsten oxide (WOs), ruthenium oxide (RuO₂), iridium oxide, cerium oxide (CeO₂), and polyoxometalate, or a mixture of one or more thereof.

In addition, metal hexacyanoferrate may be used as the electrode coating material. The metal hexacyanoferrate may include at least one of, for example, prussian blue, iron hexacyanoferrate (FeHCF), copper hexacyanoferrate (CuHCF), and cobalt hexacyanoferrate (CoHCF), or a mixture of one or more thereof.

The electrode coating material and base material describe above may be formed in a structure in which multiple layers are stacked. The electrode coating material may be coated by using at least one of drop-coating, dip-coating, spin-coating, spray coating, printing, pyrolysis, and electrodeposition.

The coated first electrode 120 and second electrode 120 may each have a surface of the coated electrode material smoothly processed by a thermocompression bonding or decal transfer method.

In addition, as illustrated in FIG. 5, the first isolation unit 140 and the second isolation unit 150 may be stacked on and coupled to the pressure generating unit 100.

The first isolation unit 140 is coupled to one side of the pressure generating unit 100, and the second isolation unit 150 is coupled to the other side of the pressure generating unit 100 in a stack structure. The first isolation unit 140 and the second isolation unit 150 are coupled to both sides of the pressure generating unit 100 to seal the pressure generating unit 100.

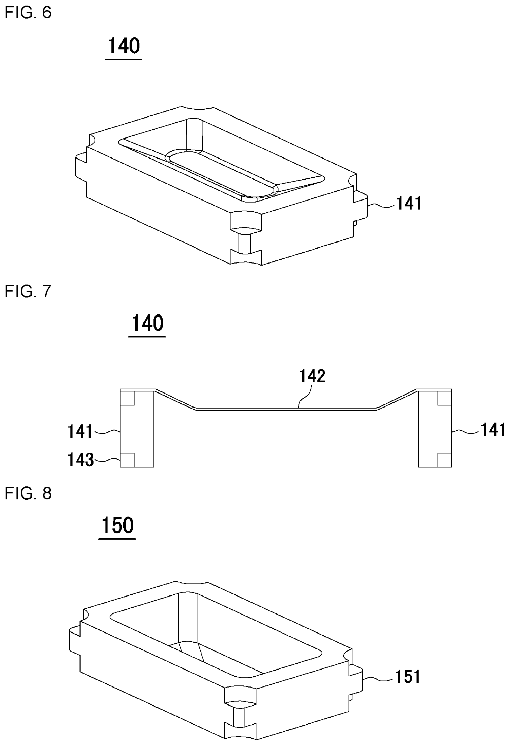

Structures of the first isolation unit 140 and the second isolation unit 150 are described below with reference to FIGS. 6 to 10.

As illustrated in FIGS. 6 and 8, the isolation unit 140 and 150 may respectively include isolation member frames 141 and 151 stacked on and coupled to the pressure generating unit 100 and the flexible isolation units 142 and 152 respectively coupled to the inside of the isolation member frames 141 and 151 to seal the pressure generating unit 100.

The first isolation unit 142 and the second isolation unit 152 may each be formed of a deformable material to be changeable in shape according to positive and negative pressures generated by the pressure generating unit 100. Accordingly, the first isolation unit 142 and the second isolation unit 152 may correspond to a diaphragm of which shape changes according to pressure.

In one example, the first isolation unit 142 and the second isolation unit 152 may each include at least one of a polymer diaphragm and a metal diaphragm. The polymer diaphragm may include at least one of, for example, a multilayer film including polyethylene (PE), polypropylene (PP), polyimide (PI), Teflon, latex, polyurethane (PU), silicone, or ethylene vinyl alcohol (EVOH), polyvinylidene fluoride (PVDF), and polyvinylidene chloride (PVDC), or a mixture of one or more thereof.

In one example, the first isolation member 142 and the second isolation member 152 may each include one or more of aluminum, stainless steel, silver, and titanium, or a mixture of one or more thereof.

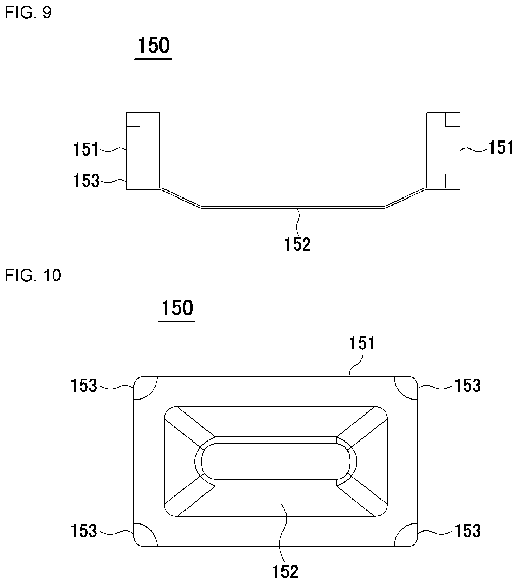

FIG. 7 and FIG. 9 illustrate cross-sectional views of the first isolation unit 140) and the second isolation unit 150.

Referring to FIGS. 7 and 9, in the first isolation unit 140, the first isolation member 142 is coupled to an upper portion of the first isolation member frame 141. In the second isolation unit 150, the second isolation member 152 is coupled to a lower portion of the second isolation member frame 151.

The isolation member frames 141 and 151 and the isolation members 142 and 152 may be coupled to each other by using at least one of a thermal fusion method, an ultraviolet (UV) bonding method, and an ultrasonic fusion method. However, the embodiment is not limited thereto, and various methods for coupling materials may be used.

As illustrated in FIG. 7 and FIG. 9, the flexible isolation members 142 and 152 may each have a length greater than internal lengths of the isolation member frames 141 and 151 such that deformation corresponding to pressure may easily occur. The internal lengths of the isolation member frames 141 and 151 may respectively correspond to diameters of hollow spaces formed inside the isolation member frames 141 and 151. That is, the first isolation member 142 and the second isolation member 152 may each have an elongated shape. Accordingly, deformation easily occurs in response to positive and negative pressures generated by the pressure generating unit 100.

In addition, the flexible isolation members 142 and 152 may each have a shape corresponding to a shape of the transfer chamber 320 or the monitoring chamber 520 when respectively bonded to the isolation member frames 141 and 151. Accordingly, a jamming phenomenon of the isolation members 142 and 152 may be prevented and a transfer efficiency of the first fluid may be increased.

FIG. 6 and FIG. 9 illustrate that each of the first isolation member frame 141 and the second isolation member frame 151 has a rectangular shape and hollow portions of the first isolation member frame 141 and the second isolation member frame 151 are also formed in a rectangular shape. However, embodiments of the present disclosure are not limited thereto, and the first isolation member frame 141 and the second isolation member frame 151 may be configured in various shapes such as circular and polygonal shapes depending on a shape of the pressure generating unit 100, a method of manufacturing method a frame, the purpose of use of the fluid transfer pump device 1, and so on.

The first isolation unit 140 and the second isolation unit 150 may be coupled to the pressure generating unit 100 to seal the pressure generating unit 100. Accordingly, the first fluid which is a transfer target fluid and the second fluid stored in the electroosmotic pump may be prevented from being mixed to each other.

In addition, the flexible isolation members 142 and 152 change in shape in response to the pressure generated by the pressure generating unit 100. The pressure generated by the pressure generating unit 100 due to a change in shape of the flexible isolation members 142 and 152 is transferred to the transfer chamber unit 300, the transfer path unit 400, and the monitoring unit 500.

In addition, as illustrated in FIG. 10, the isolation member frames 141 and 151 may respectively include one or more coupling grooves 143 and 153 to be aligned and coupled to one or more of the pressure generating unit 100, the transfer chamber unit 300, and the monitoring unit 500. The coupling grooves 143 and 153 may be coupled to coupling grooves formed in the pressure generating unit 100, the transfer chamber unit 300, and the monitoring unit 500 to form an aligned coupling structure.

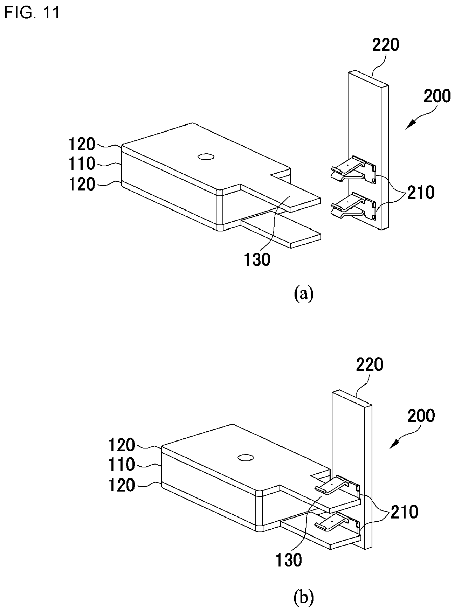

Hereinafter, a configuration of the power supply unit 200 will be described with reference to FIGS. 11 to 15.

FIG. 11 is example views of coupling of the pressure generating unit 100 and the power supply unit 200.

FIG. 11(a) illustrates a state before the pressure generating unit 100 is coupled to the power supply unit 200. FIG. 11 (b) illustrates a state in which the pressure generating unit 100 is slidably coupled to the power supply unit 200. The power supply unit 200 includes one or more connectors 210 and a support member 220 that fixes and supports the connectors 210 to be coupled to the terminal portions 130. The connectors 210 may each have a clip or clamp shape. In addition, a distance between the connectors 210 is set to correspond to a distance between the first electrode 120 and the second electrode 120.

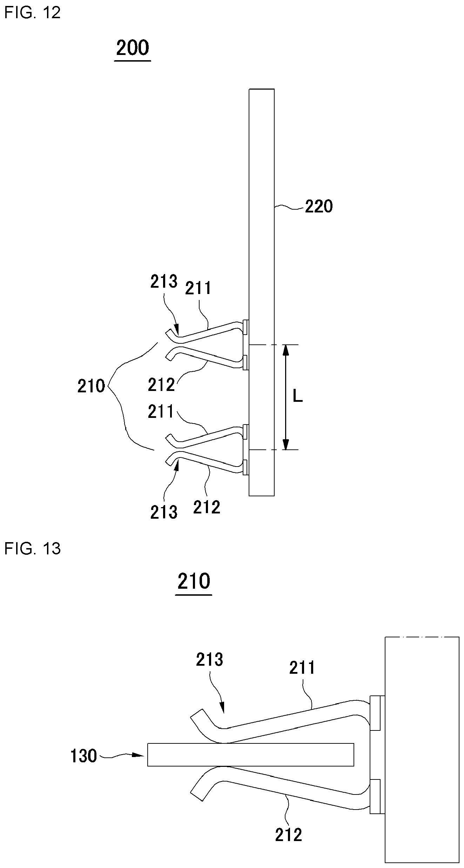

As illustrated in FIG. 12, the connectors 120 (may be referred to as a first connector 210 and a second connector 210) may be fixed to the support member 220 at a preset distance L. The distance L between the connectors 210 may correspond to a distance between the electrodes 120 or the terminal portions 130. That is, the distance L between the first connector 210 coupled to the first electrode 120 and the second connector 210 coupled to the second electrode 120 may correspond to a distance between the first electrode 120 and the second electrode 120 or a distance between the terminal portion 130 of the first electrode 120 and the terminal portion 130 of the second electrode 120. Accordingly, the first electrode 120 and the second electrode 120 may be respectively and slidably connected to the first connector 210 and the second connector 210 without separate manipulation.

The connectors 210 may each include a first leg portion 211 and a second leg portion 212 formed to elastically support both sides of the terminal portion 130. In addition, the first leg portion 211 and the second leg portion 212 may respectively have folded portions 213 formed to expand an interval therebetween toward a side where the terminal portion 130 is coupled.

As the interval of the side where the folded portions 213 are coupled to the terminal portion 130 is expanded, even when there is an error of the distance L between the connectors 210 or a position of the terminal portion 130, the terminal portion 130 and the connector 210 may be easily and slidably connected to each other.

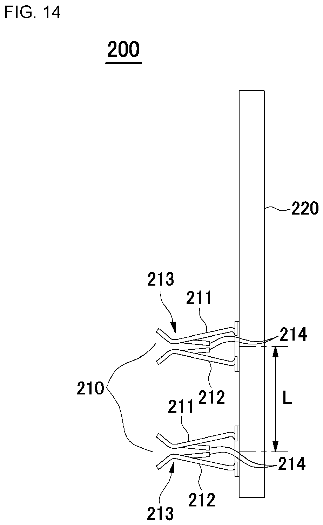

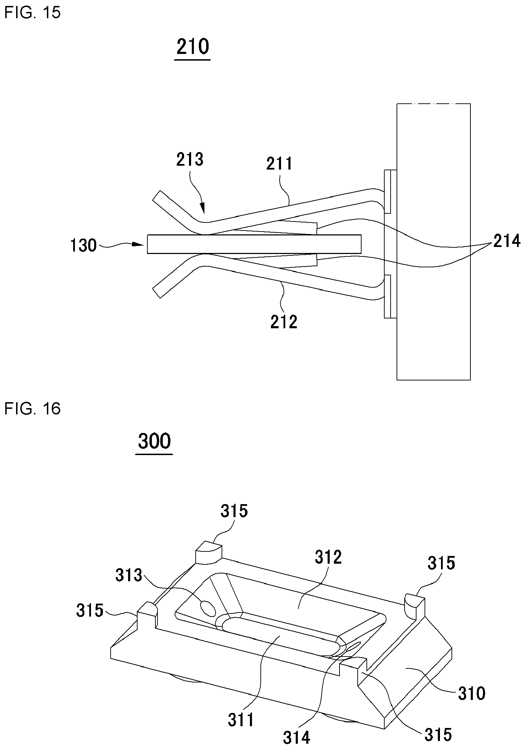

As illustrated in FIG. 13, a part of the connector 210 comes into contact with the terminal portion 130 as the interval between the first leg portion 221 and the second leg portion 222 decreases. Accordingly, the connector 210 may be deformed into a shape illustrated in FIG. 14 and FIG. 15 to increase a contact area with the terminal portion 130.

As illustrated in FIG. 14 and FIG. 15, the connector 210 may further include a third leg portion 214 that is bent from at least one of the first leg portion 211 and the second leg portion 212 toward the terminal portion 130 to expand a contact surface with the terminal portion 130.

As illustrated in FIG. 15, when the electrode 120 is coupled to the connector 210, not only the first leg portion 211 and the second leg portion 212 but also the third leg portion 214 come into contact with the terminal portion 130. Accordingly, a contact area of the connector 210 and the terminal portion 130 is increased by an area of the third leg portion 214.

However, although FIGS. 14 and 15 illustrates that the third leg portion 214 has a rectangular shape, the third leg portion 214 is not limited thereto and may be changed to various shapes depending on a shape of the terminal portion 130, a method of producing the power supply unit 200, and a method of coupling the power supply unit 200 to the terminal portion 130.

In addition, the connectors 210 are not limited to the clip shape. A power supply terminal may have not only a clamp type, but also a shape in which an electrode is attached to the electrode 120 by using magnetism, or a shape in which external pressure is applied to upper and lower portions of the electrode 120 to fix the electrode 120 or form a contact surface with the electrode 120.

The connectors 210 may be automatically assembled. For example, the connectors 210 may be packaged in a reel type which is one of packaging methods for automatic assembly. In addition, the connectors 210 may be formed to have a surface mounting device (SMD) shape. As the connectors 210 are configured in the SMD shape, the connectors 210 may be designed to have a shape that may be directly mounted on a surface of a power circuit board. By packaging the connectors 210 in a reel type and manufacturing the connectors 210 in the SMD shape, production costs may be reduced and the assembly time may be shortened.

The power supply unit 200 applies voltages to the first electrode 120 and the second electrode 120. In this case, there is a voltage difference between the voltages applied to the first electrode 120 and the second electrode 120. Accordingly, the first electrode 120 and the second electrode 120 undergo a redox reaction due to the voltage difference.

The redox reaction of the first electrode 120 and the second electrode 120 causes imbalance in electric charges, and accordingly, positive ions move through surfaces of the membrane 110 inside the first electrode 120 and the second electrode 120 to balance the electric charges. One of the first electrode 120 and the second electrode 120 generates positive ions due to an electrochemical reaction, and the other consumes the positive ions. In this case, the positive ions generated or consumed due to the electrochemical reaction may be monovalent cations. The positive ions are not limited to the monovalent cations and may include various ions, such as hydrogen ions (H⁺), sodium ions (Na⁺), and potassium ions (K⁺)

The positive ions slide across the surfaces of the membrane 110 charged with negative charges by the voltages applied to both ends, and the hydrated water molecules and the water molecules coupled to the hydrated water molecules by hydrogen bonds are coupled to each other, causing the electroosmotic pump to move the second fluid at a high speed.

As described above, the pressure generated by the pressure generating unit 100 causes deformation of the second separation member 152. According to the deformation of the second isolation member 152, the pressure generated by the pressure generating unit 100 is transferred to the transfer chamber unit 300.

Hereinafter, a structure of the transfer chamber unit 300 will be described with reference to FIGS. 16 to 21.

The transfer chamber unit 300 includes the transfer chamber frame 310 defining an outer shape and the transfer chamber 320 formed concavely in the transfer chamber frame 310 facing the second isolation member 152.

The transfer chamber frame 310 has a concave surface coupled to the second isolation member 152 to constitute the transfer chamber 320. Accordingly, the transfer chamber frame 310 includes a bottom portion 311 forming a concave space and a peripheral portion 312 surrounding the bottom portion 311. In this case, a cross-sectional area decreases from an upper portion (second isolation member) of the peripheral portion 312 toward the bottom portion 311. Accordingly, the peripheral portion 312 forms an inclined surface.

In addition, the peripheral portion 312 includes an inlet 313 to which the first fluid is input and an outlet 314 through which the first fluid is output. That is, the inlet 313 and the outlet 314 may each be formed on one of the inclined surfaces. In addition, the inlet 313 and the outlet 314 are formed to face each other in the peripheral portion 312. That is, the inlet 313 and the outlet 314 may be formed in surfaces facing each other among a plurality of inclined surfaces. Accordingly, the input and output of the first fluid may be separately and smoothly performed.

In this case, the inlet 313 and the outlet 314 are referred to for the sake of convenience of description, and are formed in the peripheral portion 312 forming the transfer chamber 320 other than an inlet where the first fluid is sucked, and mean holes coupled to the inflow path 330 and the outflow path 340.

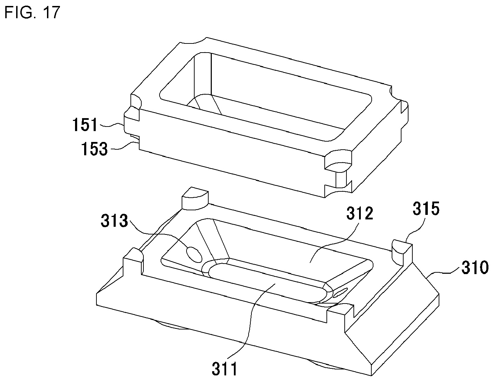

Referring to FIGS. 17 to 18(c), the transfer chamber unit 300 is coupled to the second isolation unit 150. The transfer chamber 320 may correspond to a space to which pressure is transferred in response to deformation of the second isolation member 152 or a space to which the first fluid moves. Accordingly, the first fluid, which is a transfer target fluid, may be input and output through the transfer chamber 320.

The transfer chamber 320 includes an upper portion that is open toward the second isolation member 152 and is sealed by the isolation member 152, a bottom portion 311 formed on an opposite side of the isolation member 152, and the peripheral portion 312 formed between the upper portion and the bottom portion. That is, the upper portion is sealed by the second isolation member 152, and the transfer chamber 320 corresponds to a space formed by the bottom portion 311 and the peripheral portion 312 that is concavely formed in the transfer chamber frame 310.

A cross-sectional area of the upper portion of the transfer chamber 320 is formed to be greater than a cross-sectional area of the bottom portion 311. Accordingly, the peripheral portion 312 is formed to be inclined.

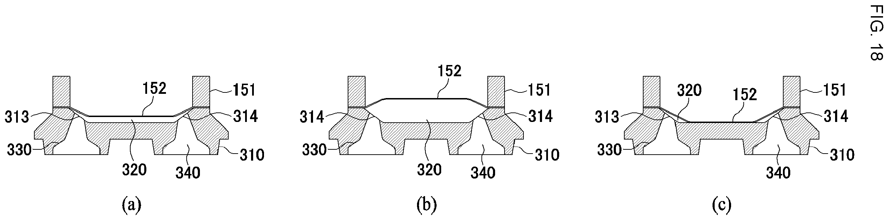

FIG. 18 is examples of deformation of the second isolation member 152.

FIG. 18(a) illustrates a shape of the second isolation member 152 when no pressure is generated by the pressure generating unit 100. When no pressure is generated by the pressure generating unit 100, the second isolation member 152 has a shape that is stretched in the direction of gravity. That is, when no pressure is generated, the second isolation member 152 has a shape that is bent toward the transfer chamber 320 or the bottom portion 311.

FIG. 18(b) illustrates a shape of the second isolation member 152 when negative pressure is generated by the pressure generating unit 100. When the negative pressure is applied to the second isolation member 152, the second isolation member 152 is deformed toward an opposite side of the bottom portion 311. According to the deformation of the second isolation member 152, the negative pressure generated by the pressure generating unit 100 is transferred to the transfer chamber 320. That is, in response to the negative pressure, the second isolation member 152 is deformed in a first shape, and accordingly, the first fluid is input to the transfer chamber 320 through the inflow path 330.

The first shape may correspond to at least one of a parabolic shape in which the second isolation member 152 is bent upward, and a shape in which the second isolation member 152 is bent into the second isolation member frame 310. In addition, the first shape may correspond to at least one of a shape in which the second isolation member 152 is bent in a direction of the pressure generating unit 100 and a shape in which the second isolation member 152 is bent in an inflow direction of the first fluid.

FIG. 18(c) illustrates a shape of the second isolation member 152 when positive pressure is generated by the pressure generating unit 100. When the positive pressure is applied to the second isolation member 152, the second isolation member 152 is deformed toward the bottom portion 311 in response to a shape of an inclined surface. Accordingly, the positive pressure generated by the pressure generating unit 100 is transferred to the transfer chamber 320 according to the deformation of the second isolation member 152. That is, the second isolation member 152 is deformed in a second shape in response to the positive pressure, and accordingly, the second fluid is output from the transfer chamber 320 through the outflow path 340.

The second shape may correspond to at least one of a parabolic shape in which the second isolation member 152 is bent toward the bottom portion and a shape in which the second isolation member 152 is bent outward from the second isolation member frame 151. In addition, the second shape may correspond to at least one of a shape in which the second isolation member 152 is bent in an opposite direction of the pressure generating unit 100 and a shape in which the second isolation member 152 is bent in an outflow direction of the first fluid.

In addition, as illustrated in FIG. 18(c), when the second isolation member 152 is deformed to the second shape, the second isolation member 152 has a shape that is bent more than the basic shape of the second isolation member 152 illustrated in FIG. 18(a). That is, when the positive pressure is generated, the second isolation member 152 is formed with a greater strain rate toward the bottom portion 311 than the basic shape.

Even when the second isolation member 152 is deformed to the second shape, there may be a gap between the second isolation member 152 and the inlet 313 and the outlet 314 because the inlet 313 and the outlet 314 are formed in the inclined surfaces. Accordingly, even when the positive pressure is generated, the first fluid may move between the inflow path 330 and the outflow path 340 through the transfer chamber

(320). In addition, because the inlet 313 and the outlet 314 are formed in the inclined surfaces, bubbles generated when the first fluid moves through the transfer chamber 320 may be smoothly discharged through the outflow path 340.

When the peripheral portion 312 of the transfer chamber 320 is formed as a vertical surface that does not include an inclined surface, the second isolation member 152 may be stuck at the corner where the bottom portion 311 meets the peripheral portion 312. In addition, bubbles and impurities generated as the first fluid moves are not discharged smoothly, and the bubbles and impurities may accumulate at the corner. Accordingly, a transfer efficiency of the first fluid decreases.

However, when the peripheral portion 312 of the transfer chamber 320 is formed to have an inclined surface, the phenomenon of the second isolation member 152 getting stuck at the corner is reduced. In addition, the discharge of bubbles generated as the first fluid moves is made smoothly. In addition, the corner accumulation of impurities is reduced, and impurities may be smoothly discharged. Accordingly, when the peripheral portion 312 of the transfer chamber 320 is formed to include an inclined surface, a transfer efficiency of the first fluid increases.

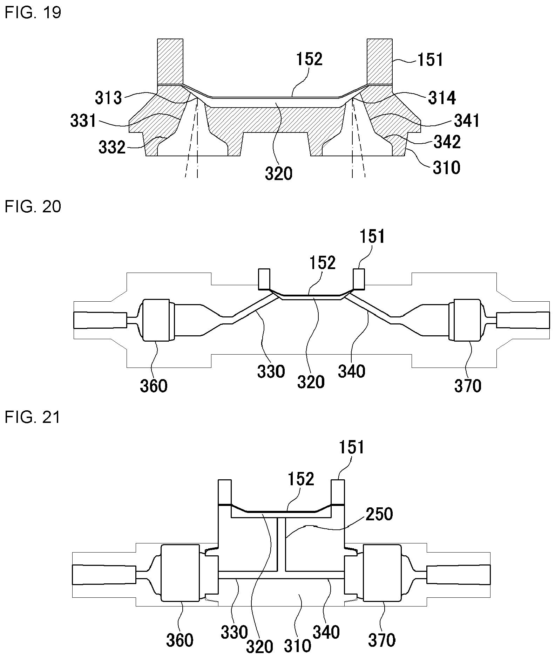

The inlet 313 and the outlet 314 formed in the peripheral portion 312 may respectively include the extended inflow path 330 and the extended outflow path 340. Specific configurations of the inflow path 330 and the outflow path 340 will be described in detail with reference to FIGS. 19 to 21.

Referring to FIG. 19, the inflow path 330 and the outflow path 340 are formed to be inclined toward the outside respectively with respect to the inlet 313 and the outlet 314. In addition, cross-sectional areas of the inflow path 330 and the outflow path 340 increase as a distance from the transfer chamber 320 increases.

That is, the inflow path 330 and the outflow path 340 may be formed to be inclined in a direction of the pressure generated by the pressure generating unit 100. In this case, the inflow path 330 and the outflow path 340 may each have an angle of 5.6° to 60°. However, angles of flow paths are not limited thereto and may be various angles depending on diameters of the flow paths, the type and capacity of the first fluid, the strength and direction of pressure, and so on.

The inflow path 330 may be divided into an upper inflow path 331 and a lower inflow path 332. The outflow path 340 may be divided into an upper outflow path 341 and a lower outflow path 342.

The upper inflow path 331 and the upper outflow path 341 are coupled to the transfer chamber 320 respectively through the inlet 313 and the outlet 314. Accordingly, one end of each of the upper inflow path 331 and the upper outflow path 341 is coupled to the peripheral portion 312 of the transfer chamber 320. The other end of each of the upper inflow path 331 and the upper outflow path 341 are respectively coupled to the lower inflow path 332 and the lower outflow path 342.

The upper inflow path 331 and the upper outflow path 341 are arranged such that flow paths are not perpendicular to a horizontal plane but form an oblique line. That is, as the inflow path 330 and the outflow path 340 are inclined outward, the upper inflow path 331 and the upper outflow path 341 are formed to be far away from each other. Accordingly, the inflow path 330 and the outflow path 340 are arranged in an A shape.

However, the embodiment is not limited thereto and may also include a case in which the inflow path and the outflow path are formed to be perpendicular or horizontal to a horizontal plane or a pressure generation direction.

In addition, the inflow path 330 and the outflow path 340 are configured to increase cross-sectional areas or diameters. The upper flow paths 331 and 341 and the lower flow paths 332 and 342 are distinguished by a difference between increase rates of the cross-sectional areas or diameters. The increase rate of the cross-sectional areas or diameters of the lower flow paths 331 and 341 may be greater than the increase rate of the cross-sectional areas or diameters of the lower flow paths 332 and 342.

That is, the inlet 313 of the upper inflow path 331 has the smallest cross-sectional area or diameter, and the cross-sectional area or diameter increases from the upper inflow path 331 toward the lower inflow path 333. The outlet 314 of the upper outflow path 341 has the smallest cross-sectional area or diameter, and the cross-sectional area or diameter increases from the upper outflow path 341 toward the lower outflow path 343.

An increase rate of the cross-sectional areas or diameters of the lower inflow path 332 and the lower outflow path 342 is greater than an increase rate of the cross-sectional areas or diameters of the upper inflow path 331 and the upper outflow path 341. However, the increase rate of the cross-sectional areas of the lower inflow path 332 and the lower outflow path 342 is not always greater than the increase rate of the cross-sectional areas of the upper inflow path 331 and the upper outflow path 341. The increase rate of the cross-sectional areas of the lower inflow path 332 and the lower outflow path 342 may be equal to the increase rate of the cross-sectional areas of the upper inflow path 331 and the upper outflow path 341 or may be configured in a straight line shape in which the cross-sectional areas do not increase, in consideration of sizes of the inflow valve 360 and the outflow valve 370 which are used, the type of the first fluid, the purpose of use, and the size of the fluid transfer pump device 1.

The lower inflow path 332 and the lower outflow path 342 are each divided into an inclined surface and a valve coupling hole. The inclined surface has a curvature. That is, the inclined surface has a streamlined shape. The valve coupling hole is configured in a shape that may be coupled to the inflow valve 360 or the outflow valve 370 depending on sizes of valves.

As described above, the flow paths 330 and 340 are arranged diagonally, and as the cross-sectional areas or diameters of the flow paths 330 and 340 increase, a vortex phenomenon is reduced even when the first fluid moves along the flow paths 330 and 340), and thus, the first fluid may smoothly flow. In addition, as the inclined surfaces of the lower flow paths 332 and 342 are each have a streamlined shape, a vortex phenomenon of the first fluid may be reduced.

However, the shapes of the flow paths 330 and 340 are not limited to the shapes in which cross-sectional areas and diameters increase while being inclined. As illustrated in FIG. 20, the flow paths 330 and 340 may be arranged diagonally and may be formed to have constant diameters. In addition, as illustrated in FIG. 21, one connection path 250 may be coupled to the transfer chamber 320, and the flow paths 330 and 340 may have a shape in which the inflow path 330 and the outflow path 340 are branched from the connection path 250.

When the flow paths 330 and 340 are arranged horizontally as illustrated in FIG. 20, the inflow path 330 and the outflow path 340 have a structure in which the inflow path 330 and the outflow path 340 are respectively coupled to both sides of the transfer chamber frame 310, not the bottom surface of the transfer chamber frame 310. That is, the lower inflow path 332 and the lower outflow path 342 are respectively arranged on the side surfaces facing each other among the side surfaces of the transfer chamber frame 310.

As illustrated in FIG. 21, when the flow paths 330 and 340 are arranged in a T shape, the inflow path 330 and the outflow path 340 are each replaced by a single T-shaped flow path without being provided individually. That is, the connection path 250, which is a central flow path of the T-shaped flow path, is coupled to the bottom portion 311 of the transfer chamber frame 310. In addition, the inflow path 330 and the outflow path 340 are branched from the connection path 250. Accordingly, when the T-shaped flow path is arranged, inflow and outflow directions of the first fluid are perpendicular to a pressure generation direction.

When the flow paths are horizontally arranged, the first fluid is sucked along the inflow path 330 by negative pressure. The sucked first fluid moves to the transfer chamber 320. When positive pressure is generated, the first fluid stored in the transfer chamber 320 moves to the outflow path 340.

When the T-shaped flow path is arranged, the first fluid is sucked along the inflow path 330 by negative pressure. The sucked first fluid moves to the transfer chamber 320 through the connection path 250. When positive pressure is generated, the first fluid stored in the transfer chamber 320 is discharged through the outflow path 340.

The inflow valve 360 and the outflow valve 370 for one-way transfer of the first fluid are coupled to the lower inflow path 332 and the lower outflow path 342.

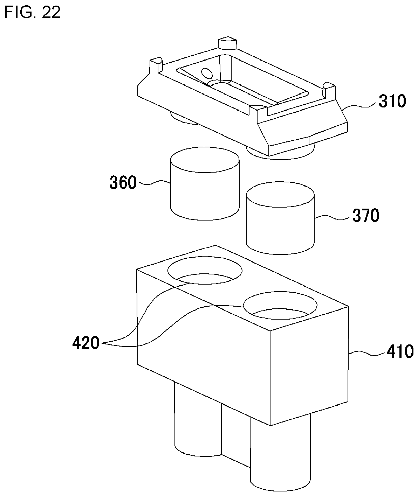

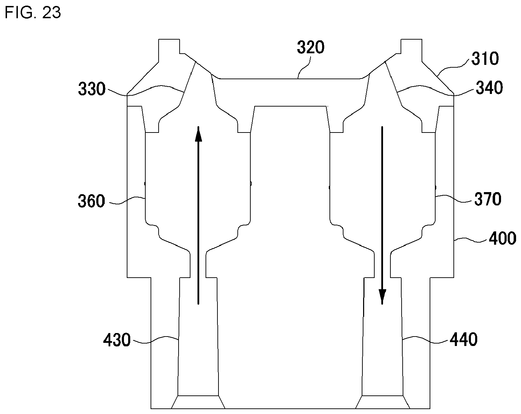

Hereinafter, structures of the inflow valve 360 and the outflow valve 370 and the transfer path unit 400 will be described with reference to FIGS. 22 and 23.

As illustrated in FIG. 22, the inflow valve 360 and the outflow valve 370 may be respectively coupled to the inflow path 330 and the outflow path 340. The inflow valve 360 and the outflow valve 370 collectively refer to a configuration for preventing backflow of the first fluid. Accordingly, the inflow valve 360 and the outflow valve 370 may each include a check valve.

The inflow valve 360 is coupled to the inflow path 330 to allow an input of the first fluid to the transfer chamber 320 and block an output of the first fluid. The outflow valve 370 is coupled to the outflow path 340 to allow the output of the first fluid to the transfer chamber 320 and block the input of the first fluid.

The transfer path unit 400 is coupled to the transfer chamber unit 300 on an opposite side of the second isolation unit 150. A pair of valve coupling holes 420, which the inflow valve 360 and the outflow valve 370 are respectively and safely placed in and fixed to, are formed in the transfer path unit 400. Accordingly, as the inflow valve 360 and the outflow valve 370 are respectively inserted into the valve coupling holes 420, the transfer chamber unit 300 and the transfer path unit 400 form a stack structure.

The transfer path unit 400 includes the inflow route 430 through which the first fluid is input to the transfer chamber unit (300) side and the outflow route 440 through which the first fluid is output from the transfer chamber unit (300) side. That is, the inflow route 430 means a path which is coupled to the inflow valve 360 or the inflow path 330 and through which the first fluid is input to the transfer chamber unit 300 side. The outflow route 440 means a path which is coupled to the outflow valve 370 or the outflow path 340 and through which the first fluid is output from the transfer chamber unit 300 side.

The inflow route 430 may include a connector connected to a storage container in which the first fluid is stored. In addition, the outflow route 440 may include a discharge device that discharges the first fluid and a connector connected to a discharge device.

When the inflow path 330 and the outflow path 340 are arranged in an A shape as illustrated in FIG. 19, the inflow route 430 and the outflow route 440 are formed in a stack direction of the fluid transfer pump device 1.

When the inflow path 330 and the outflow path 340 are arranged horizontally or in a T shape as illustrated in FIG. 20 or FIG. 21, the inflow route 430 and the outflow route 440 are perpendicular to the stack direction of the fluid transfer pump device 1.

Hereinafter, a configuration of the monitoring unit 500 will be described with reference to FIGS. 24 to 25.

The monitoring unit 500 detects a normal operation of the fluid transfer pump device 1 and blockage of the flow paths 330 and 340 of the first fluid. The monitoring unit 500 is stacked on and coupled to the first isolation unit 140 on an opposite side of the pressure generating unit 100.

The monitoring unit 500 includes a monitoring chamber frame 510 stacked on and coupled to the first isolation unit 140, a monitoring chamber 520 formed inside the monitoring chamber frame 510, and a pressure sensor 530 for measuring the pressure of the monitoring chamber 520. The pressure sensor 530 may be connected to a connection terminal 531.

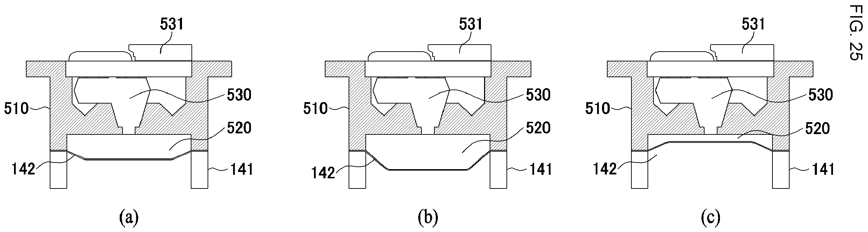

The connection terminal 531 may convert information measured by the pressure sensor 530 into an electrical signal and transmit the measured information to an external device. The connection terminal 531 is not limited to the shape illustrated in FIG. 25 and may be formed in a shape that may be directly connected to a printed circuit board (PCB).

In addition, in one embodiment, a monitoring unit control circuit may be provided instead of the connection terminal 531. The monitoring unit control circuit may determine whether the flow path is blocked and whether the fluid transfer pump device 1 operates normally, by using the pressure measured by the pressure sensor 530. In addition, when the monitoring unit control circuit detects blockage of the flow path and an abnormal operation of the fluid transfer pump device 1, the monitoring unit control circuit may generate a notification signal.

The monitoring chamber 520 may correspond to a space formed by the monitoring chamber frame 510 and the first isolation member 142. Accordingly, the pressure of the monitoring chamber 520 changes due to deformation of the first isolation member 142.

The monitoring chamber frame 510 may include one or more coupling protrusions 512 for performing aligned coupling. The coupling protrusions 512 may be coupled to the coupling grooves 143 formed in the first isolation unit 140. Accordingly, the first isolation unit 140 and the monitoring unit 500 are stacked and coupled to each other, and the pressure of the monitoring chamber 520 changes according to deformation of the first isolation member 142.

The monitoring chamber frame (510) is coupled to one surface of the first isolation member frame 141. The monitoring chamber frame 510 may be coupled to a surface symmetrical to the surface on which the pressure generating unit 100 is coupled to the first isolation member frame 141.

The monitoring chamber frame 510 includes an engraved groove or internal space such that the monitoring chamber 520 may be formed. The monitoring chamber 520 means a space in which the pressure or the shape of a space is changed according to the deformation of the first isolation member 142.

The pressure sensor 530 may be connected to the monitoring chamber 520. A pressure sensor coupling hole 511 communicating with the monitoring chamber 520 is formed in the monitoring chamber frame 510. The pressure sensor 530 is inserted into and fixed to the pressure sensor coupling hole 511. Accordingly, the pressure sensor 530 may measure a pressure change of the monitoring chamber 520.

The pressure measurement sensor 530 measures a change in pressure or shape of the monitoring chamber 520. The pressure sensor 530 may detect whether the fluid transfer pump device 1 operates normally by using the pressure change pattern measured in the monitoring chamber 520 for a certain period of time and/or a pressure average value of the monitoring chamber 520 which is measured for a certain period of time.

The monitoring unit 500 is separately provided from the flow paths 330, 340, 430, and 440 through which the first fluid moves. That is, the monitoring unit 500 determines whether the fluid transfer pump device 1 operates normally or whether the flow paths 330, 340, 430, and 440 are blocked without being directly connected to the flow paths 330 and 340 through which the first fluid moves.

FIG. 25(a) illustrates the shape of the first isolation member 142 when no pressure is generated by the pressure generating unit 100. When no pressure is generated, the first isolation member 142 has a shape that is stretched in the direction of gravity. That is, when no pressure is generated by the pressure generating unit 100, the first isolation member 142 has a parabolic shape that is bent downward or in a direction of the pressure generating unit 100.

FIG. 25(b) illustrates a shape of the first isolation member 142 when positive pressure is generated by the pressure generating unit 100. When the positive pressure is generated by the pressure generating unit 100, the first isolation member 142 is deformed to a second shape. The second shape means a shape in which the first isolation member 142 is deformed toward an opposite side of the monitoring chamber 520. Alternatively, the second shape may correspond to at least one of shapes in which the first isolation member 142 is bent into the first isolation member frame 141. In addition, the second shape may correspond to at least one of a shape in which the first isolation member 142 is bent toward the pressure generating unit 100 and a shape in which the first isolation member 142 is bent in an outflow direction of the first fluid.

FIG. 25(c) illustrates a shape of the first isolation member 142 when negative pressure is generated by the pressure generating unit 100. When the negative pressure is generated by the pressure generating unit 100, the first isolation member 142 is deformed to the first shape. As illustrated in FIG. 25(b), the first shape means a shape in which the first isolation member 142 is deformed toward the monitoring chamber 520. Alternatively, the first shape may correspond to at least one of shapes in which the first isolation member 142 is bent outward from the first isolation member frame 141. In addition, the first shape may correspond to at least one of a shape in which the first isolation member 142 is bent in an opposite direction of the pressure generating unit 100 and a shape in which the first isolation member 142 is bent in an inflow direction of the first fluid.

When the first isolation member 142 is deformed according to to the positive pressure, the first isolation member 142 has a shape that is bent more than the basic shape of the first isolation member 142 illustrated in FIG. 25(a). That is, when the positive pressure is applied to the first isolation member 142, a deformation rate of the first isolation member 142 increases. Accordingly, the first isolation member 142 has a shape that is bent (deformed) more toward an opposite side of the monitoring chamber 520 than the basic shape.

As described above, the second isolation member 152 and the first isolation member 142 are deformed in the same direction or shape in response to the negative pressure and positive pressure generated by the pressure generating unit 100.

The above descriptions of the present disclosure are for illustrative purposes only, and those skilled in the art to which the present disclosure belongs will understand that the present disclosure may be easily modified into another specific form without changing the technical idea or essential features of the present disclosure. Therefore, the embodiments described above should be understood as illustrative in all respects and not limiting. For example, each component described as a single type may be implemented in a distributed manner, and similarly, components described in a distributed manner may also be implemented in a combined form.

The scope of the present disclosure is indicated by the following claims rather than the detailed description above, and the meaning and scope of the claims and all changes or modifications derived from the equivalent concepts should be interpreted as being included in the scope of the present disclosure.

## Claims

1. A fluid transfer pump device comprising:
a pressure generating unit configured to alternately and electrochemically generate positive pressure and negative pressure;
a first isolation unit coupled to a first side of the pressure generating unit;
a second isolation unit coupled to a second side of the pressure generating unit; and
a transfer chamber unit coupled to the second isolation unit on an opposite side of the pressure generating unit.

2. The fluid transfer pump device of claim 1, wherein
the pressure generating unit, the first isolation unit, the second isolation unit, and the transfer chamber unit are stacked in one direction and integrally coupled to each other.

3. The fluid transfer pump device of claim 2, wherein
the pressure generating unit, the first isolation unit, the second isolation unit, and the transfer chamber unit are formed and aligned to have a rectangular cross-sectional outer shape with respect to a stacking direction.

4. The fluid transfer pump device of claim 1, wherein
the pressure generating unit is an electroosmotic pump.

5. The fluid transfer pump device of claim 4, wherein the electroosmotic pump includes:
a membrane configured to allow movement of a fluid; and
a first electrode and a second electrode which have a plate shape and are respectively stacked on and respectively coupled to both sides of the membrane.

6. The fluid transfer pump device of claim 1, wherein the first isolation unit includes:
a first isolation member frame stacked on and coupled to the pressure generating unit; and
a flexible first isolation unit coupled to an inside of the first isolation member frame and sealing the pressure generating unit.

7. The fluid transfer pump device of claim 6, wherein
the first isolation member frame is coupled to the first isolation member by using at least one of a thermal fusion method, an ultraviolet ray bonding method, and an ultrasonic fusion method.

8. The fluid transfer pump device of claim 1, further comprising:
a monitoring unit coupled to the first isolation unit on the opposite side of the pressure generating unit.

9. The fluid transfer pump device of claim 8, wherein
the pressure generating unit, the first isolation unit, the second isolation unit, the transfer chamber unit, and the monitoring unit are stacked in one direction and integrally coupled to each other.

10. The fluid transfer pump device of claim 8, wherein
the pressure generating unit, the first isolation unit, the second isolation unit, the transfer chamber unit, and the monitoring unit are formed and aligned to have a rectangular cross-sectional outer shape with respect to a stacking direction.

11. The fluid transfer pump device of claim 8, wherein the monitoring unit includes:
a monitoring chamber frame stacked on and coupled to the first isolation unit;
a monitoring chamber formed inside the monitoring chamber frame; and
a pressure sensor configured to measure pressure of the monitoring chamber.

12. The fluid transfer pump device of claim 11, wherein
a pressure sensor coupling groove communicating with the monitoring chamber is formed in the monitoring chamber frame, and
the pressure sensor is inserted into and fixed to the pressure sensor coupling groove.

13. The fluid transfer pump device of claim 1, wherein the second isolation unit includes:
a second isolation member frame stacked on and coupled to the pressure generating unit; and
a second isolation member that is flexible and coupled to an inside of the second isolation member frame and seals the pressure generating unit.

14. The fluid transfer pump device of claim 13, wherein
the second isolation member frame is coupled to the second isolation member by using at least one of a thermal fusion method, an ultraviolet ray bonding method, and an ultrasonic fusion method.

15. The fluid transfer pump device of claim 1, wherein the transfer chamber unit includes:
a transfer chamber frame stacked on and coupled to the second isolation unit;
a transfer chamber formed inside the transfer chamber frame to receive a transfer target fluid;
an inflow portion through which the transfer target fluid is input to the transfer chamber; and
an outflow portion through which the transfer target fluid is output from the transfer chamber.

16. The fluid transfer pump device of claim 1, wherein the transfer chamber unit includes:
a transfer chamber frame stacked on and coupled to the second isolation unit;
a transfer chamber formed inside the transfer chamber frame to receive a transfer target fluid; and
an inflow and outflow portion through which the transfer target fluid is input and output to and from the transfer chamber.

17. The fluid transfer pump device of claim 1, further comprising:
a transfer path unit coupled to the transfer chamber unit on an opposite side of the second isolation unit.

18. The fluid transfer pump device of claim 17, wherein the transfer path unit includes:
a transfer path frame stacked on and coupled to the transfer chamber unit;
an inflow route through which a transfer target fluid is input toward the transfer chamber unit; and
an outflow route through which the transfer target fluid is output from the transfer chamber unit.

19. The fluid transfer pump device of claim 18, wherein
the inflow route and the outflow route are formed in a stacking direction of the fluid transfer pump device.

20. The fluid transfer pump device of claim 18, wherein
the inflow route and the outflow route are formed to be perpendicular to a stacking direction of the fluid transfer pump device.

21. The fluid transfer pump device of claim 17, further comprising:
a valve unit configured to allow or block a flow of a transfer target fluid,
wherein the valve unit includes an inflow valve configured to allow an input of the transfer target fluid to the transfer chamber unit and configured to block an output of the transfer target fluid, and an outflow valve configured to allow an output of the transfer target fluid to the transfer chamber unit and configured to block an input of the transfer target fluid.

22. The fluid transfer pump device of claim 21, wherein
a pair of valve coupling grooves, which the inflow valve and the outflow valve are safely placed on and fixed to, are formed in the transfer path unit.

23. The fluid transfer pump device of claim 1, wherein
at least one coupling protrusion for aligned coupling and at least one coupling groove corresponding to the at least one coupling protrusion are formed in at least one of the first isolation unit and the second isolation unit, which are stacked to be coupled to each other, and the transfer chamber unit.

24. An electroosmotic pump comprising:
a membrane configured to allow movement of a fluid; and
a first electrode and a second electrode respectively provided on both sides of the membrane,
wherein the membrane, the first electrode, and the second electrode are stacked in a plate shape and coupled to each other, and
the first electrode and the second electrode respectively include electrode portions corresponding to the membrane, and terminal portions integrally extended and protruded to one side of each of the electrode portions.

25. The electroosmotic pump of claim 24, wherein
the terminal portion has a plate shape.

26. The electroosmotic pump of claim 24, further comprising:
a power supply unit connected to the terminal portions,
wherein the power supply unit includes a first connector and a second connector respectively connected to the terminal portions, and a support member fixedly supporting the first connector and the second connector.

27. The electroosmotic pump of claim 26, wherein
the first connector and the second connector are fixed on the support member at a preset distance matching a distance between the first electrode and the second electrode and are respectively and slidably coupled to the first electrode and the second electrode.

28. The electroosmotic pump of claim 26, wherein
each of the first connector and the second connector includes a first leg portion and a second leg portion formed to elastically support both sides of each of the terminal portions.

29. The electroosmotic pump of claim 28, wherein
each of the first leg portion and the second leg portion has a folded portion formed to elastically support one of the terminal portion and to expand an interval between the first leg portion and the second leg portion toward a side surface to which the one of the terminal portions is coupled.

30. The electroosmotic pump of claim 28, further comprising:
a third leg portion formed to be folded from at least one of the first leg portion and the second leg portion toward the one of the terminal portions to expand a contact surface with the one of the terminal portions.

31. A fluid transfer device comprising:
an isolation member which is flexible and to which positive pressure and negative pressure are alternately transferred; and
a transfer chamber unit coupled to the isolation member,
wherein the transfer chamber portion includes a transfer chamber frame defining an outer shape of the transfer chamber unit, and a transfer chamber which is formed concavely in the transfer chamber frame to face the isolation member and which a transfer target fluid is input to and output from.

32. The fluid transfer device of claim 31, wherein the fluid transfer chamber includes:
an upper portion that is open toward the isolation member and is sealed by being coupled to the isolation member;
a bottom portion formed on an opposite side of the isolation member; and
a peripheral portion formed between the upper portion and the bottom portion.

33. The fluid transfer device of claim 32, wherein
a cross-sectional area of the upper portion is greater than a cross-sectional area of the bottom portion, and
the peripheral portion has an inclined surface.

34. The fluid transfer device of claim 33, wherein,
when the positive pressure is applied to the transfer chamber through the isolation member, the isolation member is deformed toward the bottom portion to correspond to a shape of the inclined surface and discharges the transfer target fluid.

35. The fluid transfer device of claim 33, wherein,
when the negative pressure is applied to the transfer chamber through the isolation member, the isolation member is deformed toward an opposite side of the bottom portion and sucks the transfer target fluid.

36. The fluid transfer device of claim 33, wherein
an inlet to which the transfer target fluid is input and an outlet through which the transfer target fluid is output from the transfer chamber are formed in the peripheral portion.

37. The fluid transfer device of claim 36, wherein
the inlet and the outlet are formed to face each other in the peripheral portion.

38. The fluid transfer device of claim 36, further comprising:
an inflow path extending from the inlet; and
an outflow path extending from the outlet.

39. The fluid transfer device of claim 38, wherein
the inflow path and the outflow path are formed to be inclined to face outward respectively with respect to the inlet and the outlet.

40. The fluid transfer device of claim 38, wherein
the inflow path and the outflow path increase in cross-sectional area as a distance from the transfer chamber increases.

41. The fluid transfer device of claim 33, wherein
an inlet-outlet through which the transfer target fluid is input and output is formed in the bottom portion.

42. The fluid transfer device of claim 41, further comprising:
a connection path extending from the inlet-outlet; and
an inflow path and an outflow path formed by being branched from the connection path.

43. The fluid transfer device of claim 42, further comprising:
an inflow valve connected to the inflow path and configured to allow an input of the transfer target fluid to the transfer chamber and configured to block an output of the transfer target fluid; and
an outflow valve connected to the outflow path and configured to allow the output of the transfer target fluid to the transfer chamber and configured to block the input of the transfer target fluid.

44. The fluid transfer device of claim 31, further comprising:
a pressure generating unit configured to alternately and electrochemically generate the positive pressure and the negative pressure; and
an isolation member frame stacked on and coupled to one side of the pressure generating unit,
wherein the isolation member is coupled to an inside of the isolation member frame to seal the pressure generating unit.
